# EUROPEAN PATENT SPECIFICATION

(11) **EP 4 393 321 B1**
(45) Date of publication and mention of the grant of the patent: **02.07.2025**
(21) Application number: 24168745.8
(22) Date of filing: 22.07.2020
(51) Int. Cl.: A23L 33/115, A23L 33/19, A23L 33/00

(54) **NUTRITIONAL COMPOSITION COMPRISING MILK FAT AND IMMUNOGLOBULIN**
ERNÄHRUNGSZUSAMMENSETZUNG MIT MILCHFETT UND IMMUNGLOBULIN
COMPOSITION NUTRITIONNELLE COMPRENANT DE LA GRAISSE DE LAIT ET DE L'IMMUNOGLOBULINE

(30) Priority: 23.07.2019 EP 19187688; 20.05.2020 EP 20175745
(43) Date of publication of application: 03.07.2024
(62) Divisional of application: 20742735.2
(73) Proprietor: FrieslandCampina Nederland B.V., 3818 LE Amersfoort (NL)
(72) Inventor: VAN NEERVEN, Ruprecht Jules Joost, 6708 WH Wageningen (NL)
(74) Representative: FrieslandCampina IP Department

(56) References cited:
- WO-A1-2018/218143
- US-A- 5 744 134
- US-A1- 2013 011 415
- US-A1- 2014 296 179
- US-A1- 2015 246 100
- US-A1- 2015 305 385
- MISTY GOOD ET AL: "The human milk oligosaccharide 2'fucosyllactose attenuates the severity of experimental necrotising enterocolitis by enhancing mesenteric perfusion in the neonatal intestine", BRITISH JOURNAL OF NUTRITION, vol. 116, no. 7, 9 September 2016 (2016-09-09), UK, pages 1175 - 1187, XP055655801, ISSN: 0007-1145, DOI: 10.1017/S0007114516002944
- HESHAM M. KORASHY ET AL: "Camel Milk Modulates the Expression of Aryl Hydrocarbon Receptor-Regulated Genes, Cyp1a1, Nqo1 , and Gsta1 , in Murine hepatoma Hepa 1c1c7 Cells", JOURNAL OF BIOMEDICINE AND BIOTECHNOLOGY, vol. 2012, 1 January 2012 (2012-01-01), pages 1 - 10, XP055655852, ISSN: 1110-7243, DOI: 10.1155/2012/782642
- BRUGMAN SYLVIA ET AL: "Mucosal Immune Development in Early Life: Setting the Stage", ARCHIVUM IMMUNOLOGIAE ET THERAPIAE EXPERIMENTALIS, BIRKHAEUSER VERLAG AG, CH, vol. 63, no. 4, 11 February 2015 (2015-02-11), pages 251 - 268, XP035511159, ISSN: 0004-069X, [retrieved on 20150211], DOI: 10.1007/S00005-015-0329-Y
- YING LI ET AL: "Exogenous Stimuli Maintain Intraepithelial Lymphocytes via Aryl Hydrocarbon Receptor Activation", CELL, ELSEVIER, AMSTERDAM, NL, vol. 147, no. 3, 16 September 2011 (2011-09-16), pages 629 - 640, XP028330387, ISSN: 0092-8674, [retrieved on 20110924], DOI: 10.1016/J.CELL.2011.09.025
- CLAIRE BOURLIEU ET AL: "Infant formula interface and fat source impact on neonatal digestion and gut microbiota : Infant formula structure affects neonatal health", EUROPEAN JOURNAL OF LIPID SCIENCE AND TECHNOLOGY., vol. 117, no. 10, 10 August 2015 (2015-08-10), DE, pages 1500 - 1512, XP055726964, ISSN: 1438-7697, DOI: 10.1002/ejlt.201500025
- BERNADETTE DELPLANQUE ET AL: "Lipid Quality in Infant Nutrition", JOURNAL OF PEDIATRIC GASTROENTEROLOGY AND NUTRITION, 1 April 2015 (2015-04-01), US, pages 1, XP055344957, ISSN: 0277-2116, DOI: 10.1097/MPG.0000000000000818

## Description

### FIELD OF THE INVENTION

The invention disclosed herein relates to the field of nutritional compositions. More in particular, the invention relates to a nutritional composition comprising ruminant milk fat and ruminant immunoglobulin.

### BACKGROUND

Nutritional compositions for infants aim to resemble human milk as much as possible, as human milk is generally seen as the ideal source of nutrition for infants up to at least 6 months of age. Although infant formula have become better and better over time, there are still important differences between human milk and infant formula.

Combinations of milk fat and vegetable fat have been applied in early life nutrition products because these are necessary to obtain the right fatty acid composition of early life nutrition formula's. However, early life nutrition products with vegetable fat as the sole fat source are more common.

The intestinal tract contains the body's largest interface between a person and his or her external environment. It prevents leakage of food and microbial components into the tissues, that may give rise to inflammation and adverse reactions to food (food allergy and intolerance). The complexity of its function is obvious when thinking that at the same time the intestine must serve two opposite functions; the selective permeability of needed nutrients from the intestinal lumen into the circulation and into the internal milieu in general and, on the other hand, the prevention of the penetration of harmful entities including microorganisms, luminal antigens, food allergens, and luminal proinflammatory factors. The latter function is known as barrier function.

The gut barrier function is comprised by four major lines of defense: 1) The biological barrier, which is made up of normal intestinal flora (gut microbiota) responsible for colonization resistance; 2) The immune barrier, which is composed of gut associated lymphoid tissue (GALT), effector and regulatory T cells, IgA producing B (plasma) cells, group 3 innate lymphoid cells, and, resident macrophages and dendritic cells in the lamina propria; 3) The mechanical barrier, consisting of the mucus layer and the closed-lining intestinal epithelial cells. The epithelial cells come into the closest possible contact in the most apical part of the lateral cell membranes ("kissing points") by specific structures named "tight junctions" (TJs), which interconnect the cells and restrict the passage of ions, molecules and cells through the paracellular space, and 4) in addition to the physical barrier of mucus and the epithelial layer, a chemical barrier exists consisting of digestive secretions, antimicrobial peptides, and other cell products derived from the epithelial cells and immune cells in the underlying mucosa (cytokines, inflammatory mediators etc.).

Aged people have an increased number of intestinal infections and -complaints. This is often linked to decreased barrier function in the elderly. However, in combination with chronic disease, for example type 2 diabetes, or COPD, intestinal barrier function is compromised in elderly people (Valentini et al., 2014). This might in part explain the increased GI tract discomfort and infections seen in elderly people (Hall et al., 2005; Man et al., 2014).

In fact, a decreased function of intestinal barrier function - independent of senescence - has been noted for intestinal disorders like intestinal infection and diarrhea, celiac disease, inflammatory bowel disease, irritable bowel syndrome, (food) allergy, but also for extra-intestinal disorders like arthritis, obesity, and type 1 and 2 diabetes (Bischoff 2011; 2014; Turner 2009). The link between intestinal barrier function and chronic illnesses is at least in part linked to microbiota composition and decreased short chain fatty acid (SCFA) production. SCFA are well known to enhance barrier function in epithelial cells (D'Souza et al 2017; Bach Knudsen 2018). SCFAs are fatty acids with short aliphatic tails consisting of 1 to 6 carbon atoms. They are produced when dietary fiber, non-starch polysaccharides, like GOS are fermented in the colon.

Aryl hydrocarbon receptor (AhR) is known as the receptor for dioxin, and has been used to screen for such components in foods. This receptor may play a role in intestinal barrier function and immune surveillance in the intestine (Li 2011). AhR deficient mice have impaired barrier function and enhanced translocation of intestinal bacteria occurs (Li 2011). Ligands for AhR are typically microbiota-derived tryptophan metabolites or components found in some vegetables like cauliflower and broccoli. Some AhR ligands have been described to be present in breast milk as well.

Recently, AhR activation leading to IL-22 production was also linked to prevention of type 2 diabetes (Natividad 2018).

In (pre-) diabetes, decreases have been noted in several short chain fatty acids (SCFA), mainly butyrate, producing intestinal bacteria. Further, microbiota composition has been linked to higher blood glucose in elderly people, as well as to metabolic syndrome (Wang et al., 2012; Zhang et al., 2013).

Most of the commercially available milk of ruminants is bovine milk. Bovine milk contains many components that have immunomodulatory and antimicrobial properties. Bovine immunoglobulins (blg), in particular bovine IgG (blgG), have been studied since the 1970's for their potential effects on immunity and infection in humans.

Bovine milk, colostrum and serum contain three types of immunoglobulins: Immunoglobulin G (IgG), IgA and IgM.

In bovine milk IgG is the main isotype present, especially in colostrum, followed by IgA and IgM. IgA is present in milk and colostrum as secretory IgA (sIgA). In mature milk IgG is the dominant isotype, whereas sIgA and IgM are present at approximately 5-10 fold lower levels. The concentration of IgG in mature milk is around 200-700 µg/ml. In colostrum the IgG levels are much higher, reaching up to 50-100 mg/ml in the first days after birth.

Bovine immunoglobulins like IgG can bind to many bacteria and viruses, including human pathogens thereby offering prevention against bacterial transfer and leakage of bacterial components over intestinal epithelium, modulating the expression of epithelial tight junction proteins, and inhibits intestinal inflammation.

The potentially mitigating role of blgG in intestinal inflammatory responses associated with decreased intestinal barrier function, and effects of immunoglobulins and colostrum have been addressed in human and animal models for necrotizing enterocolitis (NEC), irritable bowel syndrome (IBS), inflammatory bowel disease (IBD), non-steroid anti-inflammatory drug (NSAID)-induced gut damage, and perioperative endotoxemia.

Immunoglobulins may be isolated or concentrated from milk or colostrum using methods known in the art (EP2280999, WO 94/13148, WO 01/03515 or from Heino et al, Functional properties of native and cheese whey protein concentrate powders, International Journal of Dairy Technology, Vol. 60, No. 4, Nov 2007, pp 277-285).

US2013/0011415 relates to milk-derived antigen specific antibodies for generating an adaptive immune response, methods of preparation, and uses thereof. It discloses a method for enhancing and/or inducing an adaptive immune response in an individual, comprising administering to said individual in need thereof an antigen-specific antibody obtained from the milk, milk product, colostrum, or colostrum product of a ruminant.

US2014/0296179 discloses a method and composition for the treatment and/or prevention of infection, said method comprising orally administering a composition to a mammal, said composition comprising a galactose containing indigestible oligosaccharide and immunoglobulin from the milk or colostrum of hyperimmunized cows. It further discloses a method of reducing the incidence of respiratory tract infection episodes comprising administering to an infant mammal in need thereof a liquid infant formula composition comprising: (a) 0.5-10 grams transgalacto-oligosaccharide per 100 grams dry weight of the composition; and (b) 0.5-10 grams fructo-polysaccharide per 100 grams dry weight of the composition.

When producing an infant formula or other milk product, legislation requires heating of the formula for safety reasons, thereby causing a denaturation of the immunoglobulins present in the formulation because immunoglobulins are heat sensitive. They start denaturing at temperatures of around 70 °C or above. At acidic pH (< 5.0) the susceptibility towards denaturation is known to be even higher.

In order to be used as an active, prophylactic ingredient in human food products, a significant amount of bovine immunoglobulins must be consumed in its intact form and must be able to survive passage through the stomach into the small intestine, or even throughout the entire gastrointestinal (GI) tract.

Surprisingly, it was found that bovine milk fat differs from vegetable fat in that it comprises other ligands that can activate the aryl hydrocarbon receptor more potently (AhR). (Activation of) the Ahr receptor is inversely associated with intestinal infection and diarrhea, intestinal bowel disease, irritable bowel syndrome, celiac disease, as well as food allergy, and non-communicable diseases as type 2 diabetes. To promote intestinal barrier function a nutritional composition comprising ruminant milk fat and immunoglobulins is provided, preferably also containing one or more of human milk oligosaccharides, prebiotics, or probiotics. Such a composition provides a two or even three fold support for the intestinal barrier function: AhR activating components present in milk fat, promote the production and excretion of antimicrobial peptides into the gut lumen b) providing functionally intact bovine immunoglobulins, like IgG, prevents infection and the occurrence of inflammation and tissue damage as a result of infection and/or leakage of bacterial components into the mucosa and optionally c) increasing the production of short chain fatty acids by the microbiota by administering non-digestible oligosaccharides or prebiotics. Preparations with bovine IgG preferably also contain other bioactive milk proteins that can influence barrier function, e.g. lactoferrin or TGF-β.

The composition of the invention is particularly useful for infants and young children as they are prone to develop infections in the gastrointestinal tract. A good intestinal barrier function is of key importance to protect them against infection. The composition of the invention is also particularly useful for elderly people with reduced gut compromised gut health or other gut-related discomforts like infections or inflammatory disorders. Additionally, or alternatively, the composition of the invention may be used to relieve the effects of type 2 diabetes.

### SUMMARY OF THE INVENTION

Accordingly, in a first aspect the invention relates to a synthetic nutritional composition comprising:
i. ruminant milk fat wherein the milk fat is capable of activating the aryl hydrocarbon receptor (AhR) in an AhR activity assay; and
ii. a ruminant immunoglobulin;
characterized in that at least 30% of the ruminant immunoglobulin is non-denatured and wherein the amount of milk fat is between 10 and 60 g of fat per 100 gram of formula, wherein the amount of ruminant fat is between 5 and 90 wt% as determined to the total amount of fat, and wherein the ruminant milk fat is not exposed to a temperature above 80 °C.

The invention further relates to the use of such a composition in a preterm formula, an infant formula, a follow-on formula or young child formula, or alternatively in an adult or elderly formulation. The use of such a composition is disclosed to treat any one or more of the indications selected from the group consisting of decreased intestinal barrier function, necrotizing enterocolitis (NEC), irritable bowel syndrome (IBS), inflammatory bowel disease (IBD), non-steroid anti-inflammatory drug (NSAID)-induced gut damage, and perioperative endotoxemia, or to treat type 2 diabetes. Similarly, such a composition is disclosed for use in the treatment of any one or more of the indications selected from the group consisting of decreased intestinal barrier function, necrotizing enterocolitis (NEC), irritable bowel syndrome (IBS), inflammatory bowel disease (IBD), non-steroid anti-inflammatory drug (NSAID)-induced gut damage, and perioperative endotoxemia; or to treat type 2 diabetes.

### DEFINITIONS

The term "treatment", in relation a given disease or disorder, includes, but is not limited to, inhibiting the disease or disorder, for example, arresting the development of the disease or disorder; relieving the disease or disorder, for example, causing regression of the disease or disorder; or relieving a condition caused by or resulting from the disease or disorder, for example, relieving, preventing or treating symptoms of the disease or disorder.

The term "prevention" in relation to a given disease or disorder means preventing the onset of disease development if none had occurred, preventing the disease or disorder from occurring in a subject that may be predisposed to the disorder or disease but has not yet been diagnosed as having the disorder or disease, and/or preventing further disease/disorder development if already present.

It is also to be understood that this invention is not limited to the specific embodiments and methods described herein, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a", "an," and "the" comprise plural referents unless the context clearly indicates otherwise. For example, reference to a component in the singular is intended to comprise a plurality of components.

It will be understood that within this disclosure, any reference to a weight, weight ratio, and the like pertains to the dry matter, in particular the dry matter of the composition, unless specified otherwise.

Unless defined otherwise, all technical and scientific terms used herein generally have the same meaning as commonly understood by one of ordinary skill in the art to which this invention belongs.

As used herein, the term "comprising", which is synonymous with "including" or "containing", is open-ended, and does not exclude additional, unrecited element(s), ingredient(s) or method step(s), whereas the term "consisting of" is a closed term, which excludes any additional element, step, or ingredient which is not explicitly recited.

As used herein, the term "essentially consisting of' is a partially open term, which does not exclude additional, unrecited element(s), step(s), or ingredient(s), as long as these additional element(s), step(s) or ingredient(s) do not materially affect the basic and novel properties of the invention.

As used herein, the term "comprising" (or "comprise(s)") hence includes the term "consisting of' ("consist(s) of'), as well as the term "essentially consisting of' ("essentially consist(s) of"). Accordingly, the term "comprising" (or "comprise(s)") is, in the present application, meant as more particularly encompassing the term "consisting of' ("consist(s) of'), and the term "essentially consisting of" ("essentially consist(s) of").

Throughout this application, where publications are referenced, the disclosures of these publications in their entireties are hereby incorporated by reference into this application to more fully describe the state of the art to which this invention pertains.

The term "subject" as used herein refers to a human. The term "subject" refers to both the male and female sex unless one sex is specifically indicated. The human subject can be an infant, a juvenile, an adolescent, an adult or an elderly subject.

As used herein, "administering a composition to a person" comprises feeding a person such as a child, baby, or infant, it also includes the person eating or drinking the composition. The preferred route of administration is oral administration.

The usual abbreviations for different units are used, for example, "mg" is used to indicate milligram, whilst "µg" indicates microgram.

As used herein, the term "synthetic nutritional composition" or a "synthetic composition" refers to a nutritional composition produced by men. A "synthetic composition" is artificially prepared and preferably means a composition comprising at least one compound that is produced ex vivo chemically and/or biologically, e.g. by means of chemical reaction, enzymatic reaction; or a product which is isolated or concentrated from a mixture of products. Concentration is defined as an increase in the wt% of the product, based on dry matter, with respect to the total composition it is in. A synthetic composition is not identical with a naturally occurring composition, i.e. it is not milk produced by a ruminant such as cow's milk, goat's milk or sheep's milk. Also, in some embodiments, the synthetic compositions may comprise one or more nutritionally or pharmaceutically active components which do not affect adversely the efficacy of the above-mentioned compounds. Some non-limiting embodiments of a synthetic composition of the invention are also described below.

The biological activity of a protein depends not only on the order of the amino acids (primary structure) but also on the way it is folded (3D structure). A "non denatured" protein, or a "functionally active" protein as used herein, means that the protein is not denatured or unfolded e.g. as a result of heat treatment, and that it can be detected by methods that discriminate between denatured and intact immunoglobulins, such as ELISA assays that can discriminate between IgG in pasteurized UHT milk powder (which is not detected), and IgG in raw milk (which is detected). A non-denatured, functionally intact ruminant immunoglobulin can be shown to bind to pathogenic bacteria or viruses as described by den Hartog et al (Den Hartog et al 2014). This distinguishes these immunoglobulins from denatured immunoglobulins that cannot bind to their respective antigens and cannot have functional activity (Ohnuki 2005). As used herein, an immunoglobulin is said to be "biologically active", my equally be referred to as the immunoglobulin being intact, or being functionally intact, in other words the immunoglobulin being non-denatured.

### DETAILED DESCRIPTION OF THE INVENTION

In a first aspect, the invention provides a synthetic nutritional composition comprising:
i. ruminant milk fat wherein the milk fat is capable of activating the aryl hydrocarbon receptor (AhR) in an AhR activity assay; and
ii. a ruminant immunoglobulin;
characterized in that at least 30% of the ruminant immunoglobulin is non-denatured, and wherein the amount of milk fat is between 10 and 60 g of fat per 100 gram of formula, wherein the amount of ruminant fat is between 5 and 90 wt% as determined to the total amount of fat, and wherein the ruminant milk fat is not exposed to a temperature above 80 °C. Preferably, at least 40% of the ruminant immunoglobulin is non-denatured, more preferably at least 50%, e.g. 60 %, particularly preferably, at least 70% is non-denatured, most preferably at least 80% is non-denatured.

The term "ruminant milk fat" as used in this connection refers to a source of milk fat from milk of ruminants, preferably bovine milk fat. The milk fat source can in principle be any available ruminant milk fat source, such as whole milk, cream, anhydrous milk fat (AMF) or milk fat fractions resulting from dry fractionation, critical CO₂ extraction or other fractionation methods known in the art. It was, however, found particularly suitable to use whole milk and/or cream as the milk fat source. Preferably the ruminant milk fat is bovine milk fat and the fat is selected from the group consisting of whole milk, cream, and anhydrous milk fat (AMF). More preferably, the bovine is a cow. In one embodiment the milk fat is bovine whole milk or cream. In one embodiment, the ruminant milk fat is bovine whole milk, in another embodiment the ruminant milk fat is bovine cream, in still another embodiment the ruminant milk fat is bovine AMF. The ruminant milk used to obtain the ruminant milk fat from, and the ruminant milk fat should not be exposed to high temperatures or acidic pH (pH< 5.0) as this will reduce the capability of activating the AhR receptor. In this context, a temperature above 80 °C is considered a high temperature, preferably the temperature does not exceed 75 °C, more preferably it does not exceed 74 °C, particularly preferably, it does not exceed 72 °C, most preferably it does not exceed 69 °C. Likewise, the time during which the milk or milk fat is exposed to an elevated temperature should be as short as possible. As such, conventional techniques can be applied to produce different milk fat sources like whole milk, cream, anhydrous milk fat (AMF) or milk fat fractions resulting from dry fractionation, critical CO₂ extraction or other fractionation methods. If a pasteurization step is needed e.g. to meet legal requirements, this is possible, but unnecessary exposure to high temperatures should be avoided and hence the milk fat or milk fat source should be kept at temperatures below 22 °C preferably below 10 °C, more preferably below 5 °C as much as possible. Alternatively, the ruminant milk fat may be obtained from fresh milk.

It is understood that the temperature and time combinations to treat the milk fat or to treat the milk from which the milk fat is derived, or any other component of the composition of the invention, should be subjected to legal pasteurization conditions if required in the application of the product of the invention, e.g. as defined in Regulation (EC) No 852/2004 of the European Parliament and of the Council of 29 April 2004 on the hygiene of foodstuffs. For example, legal pasteurization conditions include a treatment involving a temperature of at least 60 °C for at least 30 minutes or involving a higher temperature for a shorter time such as at least 72 °C for at least 15 seconds or any other combination of time-temperature conditions that result in an equivalent effect for example as can be shown by a negative phosphatase test immediately after such treatment. Such tests and pasteurization techniques are well known in the art. If the milk fat component is not yet combined with the Ig component, temperatures above 80C may be possible for a limited period (seconds) to meet regulatory requirements. Suitable conditions may be easily determined using the an AhR assay e.g. an AhR-based PAH CALUX^{®} reporter gene bioassay as disclosed in example 1.

Processes to prepare a powder e.g. a whole milk powder, are known the art for example spray drying or freeze drying for example as described in Dairy Science and Technology 2nd Edition (P. Walstra, J.T.M. Wouters and T.J. Geurts, CRC Press Taylor&Francis 2006, chapter 20. Such processes are flexible and can be used at temperatures below or above 80 °C.

The composition of the invention may be prepared by mixing the milk fraction and immunoglobulin fraction in a liquid or dry state. If admixed in a liquid state, then it may be dried using methods known in the art. The ruminant (e.g. bovine) milk fat can be applied in nutritional compositions in which between 5% and 100% of the total fat content in the composition is made of bovine milk fat, preferably between 10% and 90% of the fat in the composition is made up by milk fat, more preferably 20 to 80% of all fat in the composition is bovine milk fat, most preferably, 20 to 60% of all fat in the nutritional composition is bovine milk fat. In another embodiment the amount of bovine milk fat in the composition of the invention is between 45 and 55% of the total amount of fat (i.e. of all fat) in the composition.

As shown in the examples, milk fat has other ligands than vegetable fat to activate AhR. So in one embodiment, the composition of the invention preferably comprises a mixture of vegetable and ruminant milk fat. The amount of ruminant milk fat is below 90 wt% as compared to the total amount of fat, e.g. below 80 wt%, preferably below 65%, more preferably below 50 wt%. The amount of ruminant milk fat is at least 5 wt% as compared to the total amount of fat in the composition, e.g. at least 10 wt%, preferably at least 20 wt%, more preferably at least 30 wt%, particularly preferred the amount of milk fat is at least 40 wt% as compared to the total amount of fat in the composition. The amount of fat in the composition depends on the target population. It may be between 5 and 50 gram per 100 gram of dry composition. Preferably it is between 10 and 40 gram per 100 gram of dry composition, more preferably between 15 and 35 gram. Particularly preferably, the total amount of fat is between 10 and 40 gram per 100 gram of dry composition and the amount of ruminant fat is between 10 and 90 wt% of total fat. Even more preferably the total amount of fat is between 10 and 40 gram per 100 gram of dry composition and the amount of ruminant fat is between 20 and 80 wt% of total fat.

In still another embodiment of the composition of the invention, the amount of immunoglobulin is between 115 µg and 50 mg per gram of dry composition; and the amount of milk fat is between 10 and 60 g of fat per 100 gram of formula, wherein the amount of ruminant fat is between 5 and 90 wt% as determined to the total amount of fat.

The biological activity of the immunoglobulin may be tested using methods known in the art. In one embodiment, the biological activity of the immunoglobulin is determined in an ELISA assay that discriminates between native and denatured immunoglobulins. Such methods are commercially available (see for example https://www.bethyl.com/ at sub page product/E10-118/Bovine+IgG+ELISA+Quantitation+Set and https://www.bethyl.com/ at sub page product/E11-118/Bovine+IgG+ELISA+Kit), and such methods have been described previously in WO2008127105A1, and WO2011087364A1. Ruminant immunoglobulins are typically heat sensitive, and heat inactivation results in loss of detection in ELISA (eg. Li et al 2006, J Agric Food Chem, 54 pp 739-746; Godden et al, 2006 J. Dairy Sci, 89 pp 3476-3483; Ohnuki et al, 2005, Animal Science Journal 76 pp 283-290).

In another embodiment the biological activity of the immunoglobulin can be tested by detecting the binding of the immunoglobulin to a human pathogen or allergen. This has for example been described in detail in Den Hartog et al 2014, Ohnuki 2005, and in WO2008127105A1. The binding of immunoglobulins to human pathogens or allergens is known to reduce or disappear upon denaturation of these proteins e.g. by high temperatures.

Immunoglobulins (Igs) are sensitive to heat. So in order to keep them biologically active, milk fractions comprising Igs preferably are not exposed to a temperatures above 80 °C, preferably the temperature does not exceed 75 °C, more preferably it does not exceed 74 °C, particularly preferably, it does not exceed 72 °C, most preferably it does not exceed 69 °C. Likewise, the time during which the milk or milk fat is exposed to an elevated temperature should be as short as possible. As indicated above, the Igs fraction or the milk from which the Igs are derived, or any other component of the composition of the invention, may be subjected to legal pasteurization conditions if required in the application of the product of the invention.

The invention as disclosed herein refers to ruminant milk fat and ruminant immunoglobulins (also referred to as antibodies). Preferably said ruminant is a cow, preferably a member of the genus Bos, more preferably the ruminant is of the species Gayal, Bos frontalis (domestic gaur), Bos mutus (Yak) or Bos taurus (Domestic Cattle). In another embodiment the ruminant is a goat or a sheep, preferably a member of the subfamily Caprinae, more preferably a member of the genera Ovis or Capra. Of the genus Ovis, the species Ovis aries (Domestic Sheep) is preferred. Of the genus Capra the species Capra aegagrus hircus (the domesticated goat) is preferred. In another embodiment said ruminant is a camel, a donkey, a bufallo, a horse or a lama. The most preferred ruminant is a cow.

In one embodiment, the immunoglobulin in the composition of the invention, is selected from one or more of the group consisting of IgG, IgA, IgM, slgA, preferably wherein the immunoglobulin is IgG, more preferably IgG1 as IgG1 is most abundant in bovine milk. In another embodiment, the immunoglobulin is IgA or slgA. (s)IgA is present in high levels in human breast milk.

In bovines, colostrum is the first milk produced by a cow just before and shortly (up to 4 days) after giving birth to a calf. Colostrum contains very high levels of bovine immunoglobulins, up to 50-100 mg/ml (vs around 1 mg/ml for normal milk). IgG1 is the predominant immunoglobulin isotype in bovine colostrum and in milk. The immunoglobulins in colostrum are absorbed into the blood by calves to protect them against infection. The immunoglobulin levels in colostrum are about 50-100 fold higher than in normal milk. Another rich source of immunoglobulins is serum, for example collected when ruminants are slaughtered.

The milk fat as used in the current invention is milk fat that activates the aryl hydrocarbon receptor (AhR) in an AhR activity assay. Such reporter assays are known in the art e.g. as described in example 1 and in Pieterse et al 2013 (Pieterse et al, 2013, Environ Sci Technol. 47(20) pp 11651-9. doi: 10.1021/es403810w). Such assays can for example be accessed through BioDetection Systems BV, Amsterdam the Netherlands, alternatively, such Human Aryl Hydrocarbon Receptor (AhR) assay kits are available from Indigo Biosciences, PA, USA (https://indigobiosciences.com/ at indigo-kits-services/aryl-hydrocarbon-receptor/). In one embodiment, the activation of the aryl hydrocarbon receptor is determined using a Calux assay e.g. as described in example 1 (*vide infra*) The nutritional composition of the invention preferably comprises 115 µg - 50 mg ruminant antibody (i.e. immunoglobulin) per gram of nutritional composition. The amounts are indicated as weight ruminant antibody per weight nutritional composition in total (so including the ruminant antibody). The nutritional composition preferably comprises 200µg-20 mg ruminant antibodies per gram of nutritional composition, more preferably 0.2-5 mg ruminant antibodies per gram of nutritional composition. The ranges are for the dry weight of the composition.

Watery nutritional compositions typically have a lower amount of ruminant antibody per amount of liquid composition. So-called "liquid formula" is typically made from dry nutritional composition by dissolving powder in water. The ratio is typically 16.5 gram powder in 90 ml water to obtain a 100 ml liquid formula.

Watery nutritional compositions of the invention thus comprise 0.16 µg/ml - 8.3 mg/ml ruminant antibodies. The amounts are indicated as weight ruminant antibody per ml of the nutritional composition in total (so including the ruminant antibody). The watery nutritional composition preferably comprises 33 µg/ml - 3.3 mg/ml ruminant antibodies, preferably 33 µg/ml - 0.8 mg/ml ruminant antibodies in the reconstituted nutritional composition. The amount of antibody in the reconstituted composition refers to the total amount of ruminant antibody in the composition after it has been mixed with a liquid like water in accordance with the instructions provided with the composition. In determining the antibody content, a composition is said to be watery when it contains at least 75% water with respect to the weight of the total composition (w/w). This is irrespective of the use of gelling agents or the viscosity of the composition.

According to the present invention, a dry product or a product in a dry state comprises at least 70 wt% dry matter, more preferably at least 73 wt% dry matter, or 75 wt% dry matter, more preferably at least 77 wt% dry matter, or 80 wt% dry matter, more preferably at least 82 wt% dry matter or 85wt% dry matter, more preferably at least 87wt% dry matter, or 90wt% dry matter, and most preferably at least 92wt% dry matter or 95wt% dry matter or even more than 98wt% dry matter. In one embodiment, the synthetic composition of the invention is a dry product.

In still another embodiment the amount of immunoglobulin in the nutritional composition of the invention is between 115 µg and 50 mg per gram of dry composition; and the amount of milk fat is between 5 and 90 wt% of the total amount of fat in the composition.

The composition may also contain one or more prebiotic ingredients known in the art. Examples of suitable prebiotics are fructo and/or galacto-oligosaccharides, with short or long chains, inulin, fucose-containing oligosaccharides, beta glycans, carob flour, gums, pectins, sialyloligosaccharides, sialyllactose, galactans with short or long chains, glucosaminogalactans and other glucosamine containing oligosaccharides and nucleotides. In a preferred embodiment, the composition of the invention comprises sialyllactose and GOS. Optionally, the composition may further comprise human milk oligosaccharides and / or non-digestible oligosaccharides to induce a microbiota that produce short chain fatty acids (SCFA). These SCFA are important to promote barrier function and also to prevent (infection-related) inflammation in the intestinal mucosa. So in another embodiment, the composition of the invention further comprises one or more oligosaccharide selected from the group consisting of human milk oligosaccharides (HMO) and non-digestible oligosaccharides. In one embodiment the amount of HMO is between 0.01 and 5.0 gram per 100 gram of composition (dry weight), preferably between 0.1 and 4.0 gram per 100 gram of composition. In another embodiment, the amount of non-digestible oligosaccharide is between 0.1 and 25 gram per 100 gram of dry composition, preferably between 1 and 20 gram, more preferably between 2 and 15 gram. In yet another embodiment, the amount of HMO is between 0.01 and 5.0 gram per 100 gram of composition and the amount of non-digestible oligosaccharide is between 0.1 and 25 gram per 100 gram of dry composition.

Human milk oligosaccharides (HMOs) are a key constituent of human milk. They are a structurally and biologically diverse group of complex indigestible carbohydrates. To date, more than 200 different oligosaccharides have been identified, varying in size from 3 to 22 monosaccharide units. The most common HMOs are the neutral fucosylated and non-fucosylated oligosaccharides. The quantity and structure of these HMOs differs significantly among women and is dependent upon Secretor and Lewis blood group status (L. Bode, J. Nutr. 136: 2127-2130, 2006.). Fucosylated HMOs were found to be the most prominent component (~77%), while sialylated HMOs accounted for about 16% of the total abundance of HMOs. The fucosylated HMOs are neutral molecules, while the sialylated HMOs are acidic. In one embodiment, the composition of the invention comprises one or more HMOs.

The HMOs of human milk are composed of various monosaccharides, namely glucose, galactose, fucose, N-acetylglucosamine and sialic acids (N-acetylneuraminic acid). The sugar fucose is an unusual molecule in that it has the L-configuration, whereas the other sugar molecules in the body have the D-configuration. The structure of HMOs is a lactose unit which may be elongated at the non-reducing-end with one or more galactose and / or N-acetylglucosamine residues (core structure). The HMO core structure may be decorated with one or more fucose residues (i.e. fucosylated HMO) and with one or more sialic acid units (i.e. sialylated HMO). A HMO may also be fucosylated and sialylated. In one embodiment, the HMO in the composition of the invention is selected from one or more of the group consisting of core HMO, sialylated HMO, and fucosylated HMO. In human milk, the most abundant HMO is 2'-fucosyllactose (a neutral trisaccharide composed of L-fucose, D-galactose, and D-glucose units, linked Fuc(α1-2)Gal(β1-4)Glc; CAS Nr 41263-94-9), with a concentration of about 2 g/l (Adams et al; 2018, Nutrafoods pp 169 - 173). Preferred HMOs are 3'-Sialyllactose (3'SL); 6'-Sialyllactose (6'SL); 2'- Fucosyllactose (2'FL); 3-Fucosyllactose (3-FL); lacto-N-tetraose (LNT), lacto-N-neotetraose (LNnT) and disialyllacto-N-tetraose (DSLNT); these are preferred HMOs. Particularly preferred nutritional compositions include at least 2'FL. In yet another embodiment the composition of the invention comprises at least 0.05 gram of 6SL per 100 gram of composition (dry weight). In one embodiment the composition of the invention comprises at least 0.05 gram of LNT per 100 gram of composition (dry weight). In still another embodiment the composition of the invention comprises at least 0.05 gram of LNnT per 100 gram of composition (dry weight). HMOs can be obtained using methods known to those of skill in the art. For example, HMOs can be purified from human milk. Individual HMOs can be further separated using methods known in the art such as capillary electrophoresis, HPLC (*e.g.,* high-performance anion-exchange chromatography with pulsed amperometric detection; HPAEC-PAD), and thin layer chromatography. See, *e.g.,* U.S. Patent Application No. 2009/0098240. Alternately, enzymatic methods can be used to synthesize HMOs. Another method to manufacture HMO's is via biosynthesis in engineered bacteria. For example, a method of preparing 2'-FL is disclosed in WO 2012/112777. Alternatively, 2'-FL is commercially available.

In one embodiment the amount of HMO in the composition of the invention is between 0.01 and 5.0 gram per 100 gram, preferably between 0.1-2 gram per 100 gram of composition (dry) or in another embodiment, when the composition is a liquid, the amount of HMO is between 0.01 and 5.0 gram per 100 mL, preferably between 0.1-2 gram per mL of composition.

In other embodiments, the composition of the invention comprises a one or more sialyloligosaccharides, preferably the sialyloligosaccharides are selected from one or more of the group consisting of disialyllacto-N-tetraose, 3'-sialyllactose, 6'-sialyllactose, 3'-sialyllactosamine, 6'-sialyllactosamine, 3'-sialyl-3-fucosyllactose, sialyllacto-N-tetraose a, sialyllacto-N-tetraose b, sialyllacto-N-tetraose c, disialyllactose, 3'-sialyl Lewis A, 3'-sialyl Lewis X, disialyllacto-N-hexaose I, disialyllacto-N-hexaose II, sialyl Lea tetra, sialyllacto-N-neotetraose c, and disialyllacto-N-fucopentaose II. Sialyloligosaccharides can be separated from milk, whey or egg, or produced from components derived here from.

In another embodiment of the method according to the invention, the composition comprises 0.25 to 20 wt.% non-digestible oligosaccharides based on dry weight of the composition, preferably wherein the non-digestible oligosaccharides are selected from one or more of galacto-oligosaccharides (GOS), and fructo-oligosaccharides (FOS), more preferably, wherein the non-digestible oligosaccharides are galacto-oligosaccharides. In other embodiments the minimum amount of non-digestible oligosaccharides is at least 1 wt% based on dry weight of the composition, such as at least 5 wt%. In yet another embodiment, the maximum amount of non-digestible oligosaccharide is 25 wt% based on dry weight of the composition, preferably less than 20 wt%, more preferably less than 15 wt%. Major constituents in GOS are oligosaccharides comprising one or more beta-galactose units with a terminal glucose at the reducing end. The galactose units are predominantly linked in a beta 1-4 or beta 1-6 linkage, although beta 1-2 and beta 1-3 linkages may also occur. In a preferred embodiment, the composition of the invention comprises galacto oligosaccharides and the galacto oligosaccharides comprise 2'galactosyl lactose (Galβ1-2Galβ1-4Glc) and 3' galactosyl lactose (Galβ1-3Galβ1-4Glc). In another preferred embodiment, the composition of the invention comprises galacto oligosaccharides and the galacto oligosaccharides comprise 3'galactosyl lactose (Galβ1-3Galβ1-4Glc) and 6' galactosyl lactose (Galβ1-6Galβ1-4Glc). Preferably the amount of non-digestible oligosaccharide is between 0.1 and 25 gram per 100 gram of dry composition.

Certain strains of bacteria have attracted considerable attention because they have been found to exhibit valuable properties for man if ingested. In particular, specific strains of the genera Lactobacilli and Bifidobacteria have been found to be able to colonize the intestinal mucosa, to reduce the capability of pathogenic bacteria to adhere to the intestinal epithelium, to have immunomodulatory effects and to assist in the maintenance of well-being. Such bacteria are sometimes called probiotics.

Although little is known about the individual species of bacteria responsible for these beneficial activities, it is generally accepted that the bifidobacteria and lactobacilli constitute important components of the beneficial gut microflora. Bifidobacteria and lactobacilli are abundant in the intestines of breast fed but not bottle-fed children, and are well recognized as beneficial commensal bacteria. Their presence correlates with fewer gastro intestinal tract and airway infections, and many Lactobacilli and Bifidobacteria species have to date been used as probiotic bacteria. The outgrowth of these bacteria is promoted by complex indigestible carbohydrates (human milk oligosaccharides and prebiotic oligosaccharides) that are present in breast milk. Prebiotic oligosaccharides are now well established as an ingredient for infant formulas to promote the outgrowth of bifidobacteria in the intestine of infants.

Extensive studies have been carried out to identify new probiotic strains. For example, EP 0 199 535, EP 0 768 375, WO 97/00078, EP 0 577 903 and WO 00/53200 disclose specific strains of Lactobacilli and Bifidobacteria and their beneficial effects.

In one embodiment, the probiotic comprises bacteria of the genus faecalibacterium, preferably it comprises *Faecalibacterium prausnitzi.*

It has been proposed to add probiotics to infant formulae to encourage gut colonization to take place and to promote colonization with the "good" bacteria - species of Bifidobacteria and Lactobacilli - rather than the harmful bacteria - pathogens such as Clostridia, etc. So, in yet another embodiment the composition of the invention comprises a probiotic, i.e. one or more probiotic bacteria. Preferably the probiotic is a Lactobacillus strain, a Bifidobacterium strain, a Streptococcus strain, a Lactococcus strain, a Leuconostoc strain, an Enterobacteriaceae strain or an Enterococcus strain. More preferably, the probiotic is a Lactobacillus strain or a Bifidobacterium strain.

In one embodiment the Lactobacillus strain is a Lactobacillus rhamnosus or Lactobacillus paracasei species. In another embodiment the Bifidobacterium strain is a Bifidobacterium lactis, Bifidobacterium longum Bifidobacterium breve or Bifidobacterium animalis species.

In yet another embodiment, preferred probiotics are selected from one or more of the group consisting of Bifidobacterium lactis (first sold by Christian Hansen company), Streptococcus thermophilus (e.g. as provided under the name TH4 by Chr. Hansen, Denmark), Lactobacillus paracasei rhamnosus GG (ATCC 53103) (e.g. as provided by Valio Oy, Finland), and Bifidobacterium longum BB536 (e.g. as provided by Morinaga Milk Industry Co. Ltd, Japan).

In still another embodiment the composition of the invention comprises at least two different probiotics.

The probiotics in the composition of the invention are preferably present in an amount of 10⁶ to 10⁹ cfu/grams of dry product, preferably 10⁶ to 10⁶ cfu/g of dry product, and even more preferably 10⁷ to 5*10⁷ cfu/grams of dry product.

Prebiotics have a number of advantages over probiotics; principally the fact that they are not alive means that they can be processed into a much wider range of foods than can the fragile probiotics. Furthermore, prebiotics do not share the problem of probiotic survival upon ingestion by the consumer. Since prebiotics stimulate growth of bacteria that are already present in the gut, they can be seen as more "natural" additives or ingredients than probiotics, which necessitate administration of extraneous bacteria.

The nutritional composition of the present invention may further comprise lactoferrin, preferably bovine lactoferrin. Lactoferrin is an iron transport protein found in human milk with antimicrobial activity. The term "lactoferrin" as used herein includes both denatured lactoferrin and large, biologically active fragments of lactoferrin (e.g., lactoferrin fragments) and undenatured or natural lactoferrin. Lactoferrin is a glycoprotein that belongs to the iron transporter or transferrin family. It is found in bovine and other mammalian milk as a minor protein component of whey proteins. Lactoferrin contains 703 amino acids, has a molecular weight of 80 kilodaltons, and is also found in human milk. Lactoferrin concentrations in the composition of the present invention are preferably at least about 10 mg/L, preferably at least about 50 mg/L. In one embodiment, the amount of bovine lactoferrin in the composition of the invention is from 10 mg/L to 2000 mg/L, preferably from 50 mg/L to 2000 mg/L, more preferably from 100 to 1500 mg/L. Human milk, by comparison, generally contains from about 1390 to about 1940 mg/L of lactoferrin. The amounts in mg/L as used in combination with Lactoferrin refer to the amount of lactoferrin per liter of ready to consume product. Suitable sources of lactoferrin for use herein include isolates, concentrates, or extracts of mammalian milk or milk products, including human and bovine milk. Bovine milk is a preferred lactoferrin source for use herein, including enriched whey protein concentrates as described herein.

Individual sources of lactoferrin suitable for use herein include Lactoferrin FD (80% Lactoferrin), available from FrieslandCampina Ingredients, The Netherlands.

The nutritional composition of the invention, also referred to as composition of the invention, is particularly suitable for human subjects of 0 to 36 months of age, in particular infants (a person of 0-12 months of age according to the CODEX Alimentarius (CODEX STAN 72-1981), further referred to as the CODEX) and young children up to the age of 36 months. Nutritional compositions for infants are commonly referred to as infant formula. When used as infant formula, the composition as used in the various embodiments of the invention should contain the ingredients in the amounts as prescribed by the CODEX and, if needed, as prescribed by additional regulations of individual countries. Alternatively, requirements for formula for infants and young children have been described in Delegated Regulation (EU) 2016/127 of 25 September 2015 supplementing Regulation (EU) No 609/2013 and Commission Directive 2006/141/EC. An example of an ingredient list of an infant formula meeting the requirements of the EU, China and Codex can for example be found on www.frieslandcampinaingredients.com/ at app/uploads/2019/04/PDS_ELN_Essential^{®}-Start-IF-110.pdf.

Accordingly, in a preferred embodiment, the nutritional composition according to the invention for infants comprises ruminant milk fat and immunoglobulin as defined elsewhere herein and further comprises protein, carbohydrates, vitamins, minerals and trace elements and the other substances in accordance with the specifications prescribed by the CODEX and, if needed, by additional national regulations.

The composition of the invention is suitable for human subjects e.g. a young child (a person of 12-36 months of age, - also referred to as toddler) and the composition is a 'follow-on formula for young children' (FUF-YC),- such a formula may also be referred to as 'growing up milks', 'growing up formulas' or 'toddlers' milk', or alternatively it may be referred to as "young child formula". Such a formula comprises the lipid, protein, and digestible carbohydrates as described above, and may further comprise vitamins, minerals and trace elements and the other substances in accordance with the specifications prescribed by the CODEX STANDARD FOR FOLLOW-UP FORMULA (CODEX STAN 156-1987) and, if needed, by additional national regulations. Accordingly, the composition as used in the various aspects of the invention is selected from one or more of the group consisting of an infant formula, a follow-on formula and young child formula, preferably, the composition is an infant formula or a follow-on formula, more preferably an infant formula.

Pre-term infants have more problems with a leaky immature gut and /or gut barrier closure. The composition of the invention offering activation of the Ahr receptor and immunoglobulin, may hence be particularly relevant for pre-term infants as this composition supports a good gut barrier function, preferably when the composition is further comprising one or more HMO. So, in yet another embodiment, the nutritional composition of the invention is a preterm formula, i.e. a formula designed for preterm babies, i.e. babies born after a pregnancy of no more than 37 weeks.

The composition of the invention may be in any form suitable in the art, for example in one embodiment it is a ready to use liquid, or preferably, in another embodiment, it is in powder form, more preferably, a free floating powder.

The composition of the invention can be a premix, e.g. a premix which may be used as a basis for a formula like an infant formula, follow-on formula, young child formula, or preterm formula. In yet another embodiment the nutritional composition of the invention is ingredient premix, preferably an early life nutrition premix.

In yet another embodiment the composition of the invention comprises
i. between 10 and 60 g of fat per 100 gram of formula, wherein the amount of ruminant fat is between 5 and 90 wt% as determined to the total amount of fat;
ii. between 115 µg - 50 mg of ruminant immunoglobulin per gram of composition (dry weight);
iii. between 10⁶ and 10⁹ cfu of probiotic per grams of dry product;
iv. between 0.1 and 25 gram of non-digestible oligosaccharide per 100 gram of formula (dry weight);
v. between 0.01 and 5.0 gram of HMO per 100 gram of dry product;
vi. between 0.1 and 20 mg of lactoferrin per gram of dry product.

Other ingredients in the composition may for example be selected from lactose, carbohydrates, vitamins, minerals etc. In still another embodiment this composition is free of probiotics, in other words the composition comprises items i. ii. iv. v. and vi.

In another aspect, the invention relates to the use of the nutritional composition of the invention in a preterm formula, an infant formula, a follow-on formula or young child formula.

The use is disclosed of the nutritional composition of the invention to treat any one or more of the indications selected from the group consisting of decreased intestinal barrier function, necrotizing enterocolitis (NEC), irritable bowel syndrome (IBS), inflammatory bowel disease (IBD), non-steroid anti-inflammatory drug (NSAID)-induced gut damage, and perioperative endotoxemia.

The use is disclosed of the nutritional composition of the invention to treat a subject suffering from type 2 diabetes (T2D).

The treatment of a disease or indication includes administration of the composition of the invention to prevent a person from suffering from said disease or indication and / or to reduce the symptoms of a disease.

The composition of the invention may also be used in adult or elderly formulations i.e. food products for grownups, elderly people or adults. In particular in the treatment of, or prevention of type 2 diabetes, IBS or IBD. Formulations for adults or elderly persons may be in the form of premixes that can be combined with other food e.g. with milk products or yoghurt. Alternatively, it can be a ready to eat product like a pill, capsule, a bar e.g. a cereal bar or chocolate bar; a candy. Alternatively, in another embodiment the food product is liquid.

The product of the invention may be used to support intestinal barrier function in a human being, e.g for prevention or treatment of diarrhea or intestinal infections. Said use can relate to early life nutrition formulas to support barrier function in the gastrointestinal tract of premature infants, infants and very young children (0-3 yr), but can also relate to the use of nutritional formulas for humans >3 years of age for the prevention and treatment of intestinal barrier function related disorders such as inflammatory bowel disease, irritable bowel syndrome, as well as intestinal barrier function related chronic illnesses such as type 2 diabetes.

The nutritional composition of the invention can also be used in a non-therapeutic way for reducing symptoms associated with selected from the group consisting of decreased intestinal barrier function, necrotizing enterocolitis (NEC), irritable bowel syndrome (IBS), inflammatory bowel disease (IBD), non-steroid anti-inflammatory drug (NSAID)-induced gut damage, and perioperative endotoxemia. In another embodiment is could be used in a non-therapeutic way for reducing symptoms associated with type 2 diabetes.

### Legend to Figures

In Figure1 A-C the PAH CALUX analysis of spike standards (A. PCB126 standard, B. PAH-mix standard, C. I3C standard) following fractionation on a de-activated silica column is shown.

In Figure 2, the fractionated PAH CALUX activity as determined in 3 bovine milk products and 1 vegetable fat is shown (A. PCB126 standard, B. PAH-mix standard, C. I3C standard).

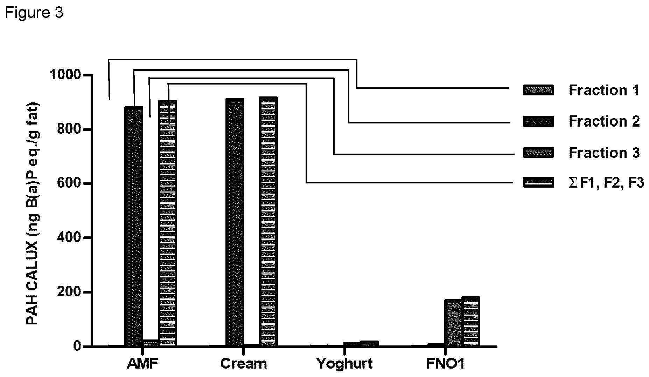

The total PAH CALUX activity (sum of PAH activity in fraction 1, 2 and 3) is shown in Figure 3. Fraction 1 is shown on the left, fraction 2 is second from left, fraction 3 is second from the right and the summation of all activity in a product is shown on the right.

AhR-ligand binding and subsequent PAH CALUX activity in AMF and cream is mainly a results of binding of metabolic instable "normal" AhR-ligands whereas the observed AhR-ligand binding and subsequent PAH CALUX activity in FN01 is mainly the result of the presence of metabolic instable "natual" AhR-ligands such as indole derivates and tryptophane metabolites. In yoghurt, only minor PAH CALUX activity was observed

### Examples

Milk cream and Anhydrous Milk Fat (AMF) were produced by FrieslandCampina Netherlands.

The milk cream had a fat% of 41.7 wt%. Milk cream and AMF were produced using methods known in the art.

Yoghurt was obtained from the supermarket (Campina Boerenland volle yoghurt, FrieslandCampina, the Netherlands).

FN01 is a mix of vegetable fats, obtained from Loders Croklaan, Wormerveer, the Netherlands. Immunoglobulins are administered as Whey Protein Concentrate (WPC), milk serum protein concentrate and/or minimally heat treated bovine milk, containing a mixture of all immunoglobulin isotypes present (FrieslandCampina Netherlands).

### Example 1: Demonstration of AhR activation by milk fat

The presence of AhR-ligands in milk fat, cream, yoghurt and FN01 a vegetable fat blend was studied using the AhR-based PAH CALUX^{®} reporter gene bioassays (BioDetection Systems BV, Amsterdam, the Netherlands ("BDS").

The PAH CALUX^{®} bioassay is an aryl-hydrocarbon receptor (AhR) transactivation bioassay comprising genetically modified H4IIE rat hepatoma cell-line, incorporating the firefly luciferase gene coupled to dioxin responsive elements (DREs) as a reporter gene for the presence of compounds able to bind the Ah-receptor. The PAH CALUX^{®} bioassay detects metabolic stable AhR-ligands (toxic AhR ligands such as dioxins, furans and dioxin-like PCBs), metabolic instable AhR-ligands (poly aromatic hydrocarbons (PAHs) and metabolizable, natural Ah-receptor ligands such as indole derivatives, tryptophane metabolites and quercetin).

Various clean-up and separation techniques were used to distinguish between non-metabolizable, toxic Ah receptor ligands (dioxins (Polychlorinated dibenzodioxins or PCDDs), furans (polychlorinated dibenzofurans or PCDFs) and dioxin-like polychlorinated biphenyls (dl-PCBs)), metabolizable, xenobiotic Ah-receptor ligands (poly aromatic hydrocarbons (PAHs)) and metabolizable, natural Ah-receptor ligands (e.g. indole derivatives, tryptophane metabolites, quercetin).

PAH CALUX^{®} cells that are exposed to compounds of interested not only express proteins that are under normal circumstances associated to the Ah-receptor activated DRE-regulated genes, but also luciferase. By addition of the appropriate substrate for luciferase, light is emitted. The amount of light produced is proportional to the amount of ligand-specific receptor binding, which is benchmarked against the relevant reference compounds (B[a]P for the PAH CALUX^{®} bioassay).

To obtain AhR-ligands from bovine milk products (milk, yoghurt, cream) and vegetable fat (FN01), the samples were extracted with hexane/DEE, 97/3 v/v. Following extraction, the extractable fat was removed using a 8% de-activated alumina column (elution 210 ml pentane) after which metabolic stable AhR-ligands (PCDD/Fs, dIPCBs), metabolic instable AhR-ligands (PAHs) and natural metabolic instable AhR-ligands (e.g. indole derivatives) were separated on a 1.5% de-activated silica column. Following elution (1: 15 ml hexane; 2: 10 ml hexane/DEE (85/15 v/v); 3: 25 ml DEE), 3 fractions were collected (fraction 1: PCDD/Fs, PCBs, apolar pesticides; fraction 2: PAHs, polar pesticides; fraction 3: indols, etc.). The extraction and separation efficiency of the combined de-activated alumina/de-activated silica column clean-up was evaluated using spiked samples containing PCB-126, PAH standards, indole-3-carbinole (I3C), 3-Indole-Acetic Acid (3IAA) and Tryptamine (TTAM). The collected fractions were evaporated and re-dissolved in DMSO.

Serial dilutions of the cleaned/separated extracts in DMSO were prepared and analyzed for Ah-receptor signaling in the PAH CALUX^{®} bioassay. In short, PAH CALUX^{®} cells were seeded in 96 wells plates. After 24 hours, cells were exposed for 4 hours (to serial dilutions of the final DMSO extracts in triplicate and then lysed to measure luciferase activities. On each 96-well plate, a complete calibration curve for each respective bioassay was also analyzed. Analysis results were interpolated in the calibration curve for determination of Ah-receptor activity. The final Ah-receptor activity is expressed as pg B(a)P equivalents per gram of processed fat.

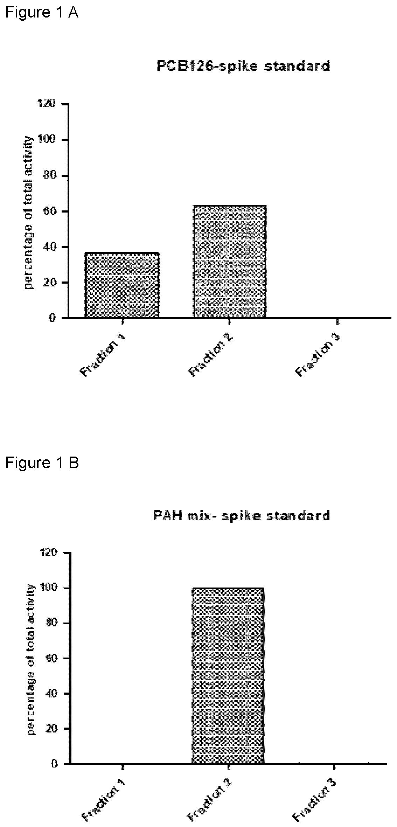

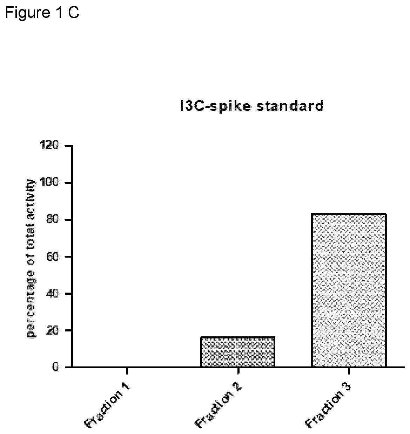

To test the efficiency of the de-activated silica fractionation, spike-standards (PCB-126, PAH-mix, I3C/3IAA/TTAM-mix) were separated on the de-activated silica column. Three fractions were collected and tested for PAH CALUX activity. In figure 1, the PAH CALUX analysis results following fractionation of the spike-standards (PCB-126, PAH-mix, indole-3-carbinole (I3C)) on a 1.5% de-activated silica column is given. Results indicate that the applied de-activated silica column separates the reference "natural" AhR-ligand I3C from metabolic stable "toxic" AhR ligands (PCB-126) and metabolic instable "normal" AhR ligands (PAH-mix). The metabolic instable "natural" AhR-ligands eluted in fraction 3 whereas the metabolic instable "normal" AhR ligands are collected in fraction 2. The metabolic stable "toxic" AhR-ligand PCB-126 was found to have eluted in both fractions 1 and 2 indicating incomplete fractionation.

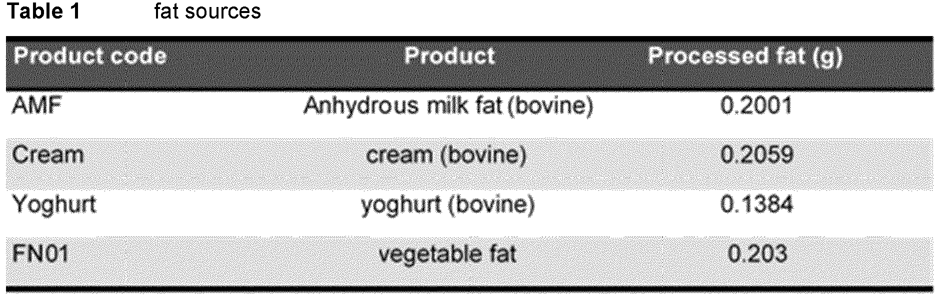

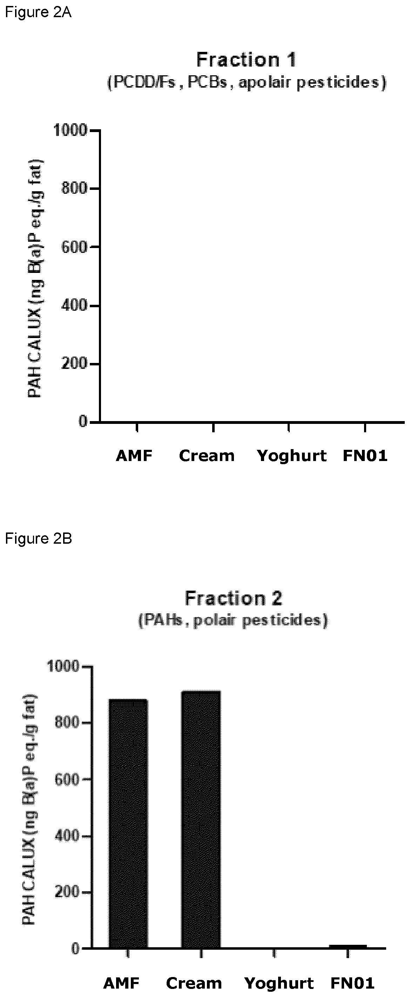

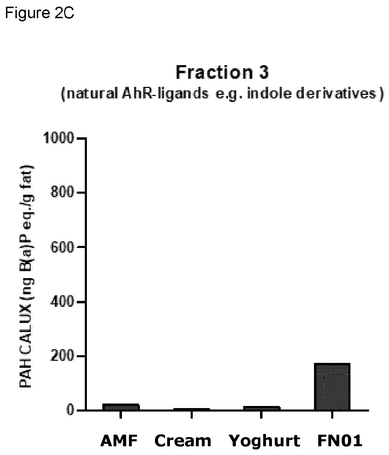

In table 1, the amount of extractable fat obtained, and processed further to determine the PAH CALUX activity of AhR-ligands in the 3 received milk products and the vegetable fat FN01 is given. For the determination of metabolically stable, "toxic", metabolically instable, PAH-like" and "metabolically instable, natural" Ah-receptor ligands, the fat was removed using a de-activated alumina columns after which the cleaned extract was fractionated using de-activated silica. The 3 final fractions obtained for each of the 4 fractionated samples were dissolved in DMSO. Serial dilutions of the final cleaned fractions in DMSO were used to expose PAH CALUX cells for4 hours. In table 2, the PAH CALUX analysis results of the fractionated milk products and vegetable fat are summarized. In figure 2, a graphical representation of the fractionated PAH CALUX analysis results of the various samples tested is given. None of the samples showed PAH CALUX activity in fraction 1 indicating the absence of "toxic" AhR-ligands such as PCDD/Fs and dIPCBs. In contrast, fraction 2 ("normal" metabolizable AhR-ligands such as PAHs) of the AMF and cream fractionated extracts showed high PAH CALUX activity whereas the yoghurt and FN01 extracts only showed minor levels of PAH activity PAH CALUX activity in fraction 3, indicating the presence of "natural" metabolizable AhR-ligand such as indole derivates and tryptophane metabolites, was observed in all samples. However, the activity in fraction of the FN01 extract was considerably higher as compared to the observed activity in the three other fractionated sample extracts.

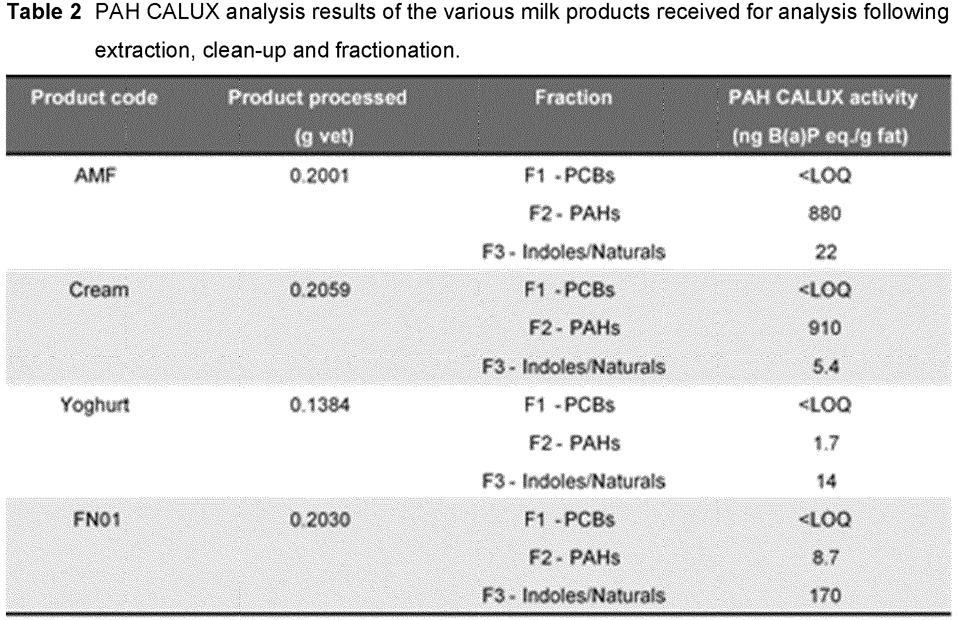

In this example it is shown that extraction of AhR-ligands from bovine milk products and vegetable fat followed by subsequent separation of metabolic stable "toxic", metabolic instable PAHs and metabolic instable "natural" AhR-ligands present in the sample extracts, clearly indicates the presence of much higher levels and different types of AhR-ligands in AMF, cream as compared to FN01 and yoghurt as determined using the PAH CALUX^{®} reporter gene bioassays. In AMF and cream, PAH CALUX activity was mainly a result of the presence of metabolic instable "normal" AhR-ligands such as PAHs. In contrast, PAH CAUX activity in FN01 was mainly caused by the presence of metabolic instable "natural" AhR-ligands such as indole derivates and tryptophan metabolites.

### Example 2

A) Human study: prevention of barrier dysfunction by nutritional composition after NSAID challenge
B) OR using ETEC as model, use composition, measure barrier function with relevant markers

### Preparation of base powder test product

Fresh whole milk and skimmed milk are standardized on protein/fat ratio and provides the active Igs. To this are added a premix minerals, a premix vitamins and lactose. Product is heat treated for 30 s at 75 C, evaporated in a MVR at 60 C, homogenized (120/30 bar) and spray dried.

### Preparation of base powder Reference product

A premix minerals, a premix vitamins + lactose are mixed with skimmed milk. The product is heated up to 80 C, kept for 2 minutes at this temperature and then heated for 5 s at 100 C in a DSI followed by evaporation in a MVR at 68 C. A vegetable fatblend is injected after evaporation, followed by homogenization (120/30 bar) and spray drying.

| **Composition of base powder (g/100 g)** | **Test** | **Reference** |
|---|---|---|
| Protein [Nx6.25] | 19,5 | 18,8 |
| Fat | 16,6 | 21,6 |
| Amount milk fat in fat | 16,4 | 0,3 |
| Lactose | 55,4 | 51,2 |
| Minerals | 4,3 | 4,2 |
| Others | 2,2 | 2,2 |

| **Blend recipes (g/100g)** | **Test** | **Reference** |
|---|---|---|
| Base powder test product | 77,1 | |
| Base powder reference product | | 83,0 |
| Galacto-oligosacharides poder (GOS) | 4,4 | 4,4 |
| Glucose syrup | 5,5 | 9,7 |
| Fatblend powder | 10,0 | 0 |
| 2'Fucosyllactose | 0,5 | 0,5 |
| Lactoferrin | 0,52 | 0,52 |
| Nucleotides, trace elements | 1.16 | 1.16 |
| Oil / minerals / vitamins | 0.81 | 0.76 |

After reconstitution of 15 g powder with 90 ml water the composition comprises:

| | **Test** | **Reference** |
|---|---|---|
| IG total [mg/100 g] | 28 | 29 |
| IG active [mg/100 g] | 21 | 0 |
| Milk fat [g/100 g] | 12,6 | 0,2 |
| Milk fat/total fat | 70% | 1% |

### Example 2A Prevention of intestinal barrier dysfunction by NSAID

To assess the effects of the nutritional composition of the invention on intestinal barrier function, a crossover study in adult volunteers is performed. The volunteers (seven healthy adults) are given the nutritional composition for a period of 1 week before ingesting indomethacin for 5 days essentially as described in (Playford et al 1999 Gut vol 44 pp 653-8).

Intestinal barrier permeability is assessed by assessing lactulose/rhamnose ratio in urine as follows. Following an overnight fast, subjects emptied their bladders and then drank a standardized sugar solution containing 5 g of lactulose, 2 g of mannitol and 1 g of rhamnose in a total of 450 ml of water (calculated osmolality 69 osmol}kg of water). Subjects were allowed unlimited intake of fluid after the first hour of the test to ensure adequate urine output. The urine was collected and pooled over the next 5 h and total volume recorded. The lactulose/rhamnose ratio is used as index of intestinal injury.

Following an initial baseline permeability assessment and collection of a baseline fecal sample, the volunteers receive, in random order, the nutritional composition (eg 18,75 gram powder dissolved in 125 ml, twice daily) or control composition for 7 days. For the last 5 days of each study arm, they also take indomethacin 50 mg, tds. At the end of the test period intestinal permeability is reassessed, and another fecal sample is collected. A 2 week 'washout' period is left between the two stages of the study. After this the volunteers undergo the same procedure, but consume the other composition, so all individuals are subjected to both the nutritional composition of the invention and the control composition.

The fecal samples are used to measure intestinal barrier function markers as measured in fecal water obtained from the fecal samples.

Fecal water extracts are prepared by adding 3.6 ml of PBS and 40µl Protease inhibitor (Sigma-Aldrich, Zwijndrecht, The Netherlands P8340) to 0,4 gram of feces, vortexing at least 30 seconds, and centrifuge at 3000x g for 10 minutes at 4°C. Fecal extracts are stored at -80°C until measurement.

To assess intestinal barrier function fecal Calprotectin (Human S100A8/S100A9), alpha-1-antitrypsin (Human SERPIN-A1) and Zonulin are measured in the fecal water extracts by ELISA according to the manufacturer's instructions (Calprotectin and alpa1-antitrypsin: R&D systems, zonulin : Alere).

In the control arm, 5 days of indomethacin intake is expected to cause a rise in lactulose/rhamnose ratios. Co-administration of the nutritional composition of the invention will result in a lower or no increase.

### Example 2B : Effect in ETEC challenge model

The effect of the nutritional composition of the invention can also be studied in an experimental ETEC infection model in adult human volunteers as described in for example ten Bruggencate 2016.

Healthy adults, aged 18-55 y, are recruited for the study and individuals who used antibiotics, immunosuppressive drugs, antacids, laxatives, or antidiarrheal drugs in the past 3 months before the study, current or previous underlying disease of the gastrointestinal tract, or lactose intolerance are excluded.

Intestinal barrier permeability is assessed by assessing lactulose/rhamnose ratio in urine as follows. Following an overnight fast, subjects empty their bladders and then drink a standardized sugar solution containing 5 g of lactulose, 2 g of mannitol and 1 g of rhamnose in a total of 450 ml of water (calculated osmolality 69 osmol/kg of water). Subjects are allowed unlimited intake of fluid after the first hour of the test to ensure adequate urine output. The urine is collected and pooled over the next 5 h and total volume recorded. The lactulose/rhamnose ratio is used as index of intestinal injury, a combination that has been recommended for assessing enteropathy.

After passing this first screening, a non-fasting blood sample is obtained. Sera are prepared and analyzed for specific IgG against the specific immunogenic epitope of diarrheagenic E. coli colonization factor antigen II (CFAII). In addition, a fresh fecal sample is obtained and analyzed for diarrheagenic E. coli by qPCR. Individuals with detectable immunoglobulin titers against CFAII, induced by previous ETEC infections, or detectable fecal diarrheagenic E. coli counts are excluded from participation in the study because of likely resistance to the E. coli strain administered in the present study. The study has 2 primary outcomes: fecal diarrheagenic E. coli excretion and fecal output. On the basis of 2-sided statistical testing for unpaired data, a = 0.05 (chance of type I error) and b = 0.20 (chance of type II error), and to compensate for dropouts, 30 participants per group should be included. Participants are stratified according to age, gender, fecal lactobacilli, and detectable immunoglobulin titers against CFAII (determined at screening) and randomly assigned to the MFGM or control group. Stratification and randomization is performed by a nonblinded person not involved in the study. The randomization code of each participant is kept in sealed envelopes, and the code was broken after finishing all laboratory and statistical analyses.

A randomized, placebo-controlled, double-blind,4-wk parallel-intervention study is performed. Participants are instructed to maintain their usual pattern of physical activity and their habitual diet throughout the entire study, but to abstain from dairy products and from products containing pre- and probiotics. Participants receive a list of dairy products and products rich in pre- or probiotics in their paper subject diary. Dairy foods are not included in the diet because they contribute substantially to total daily calcium intakes. Dairy products are replaced by providing participants with low-calcium soy drinks. During the entire study, participants consume twice daily a 250-mL soy-milk drink (e.g. Alpro- Soya Bio Nature). Drinks are consumed in the morning (125 mL) and evening (125 mL) during breakfast and dinner.

Four days before the diarrheagenic E. coli challenge and 24 hours after the diarrheagenic E. coli challenge, intestinal barrier permeability is assessed by assessing lactulose/rhamnose ratio in urine as described above.

Two days before and 2 d after the diarrheagenic E. coli challenge, participants quantitatively report all food and drinks consumed in an online nutrition diary. The weight of the food and drinks consumed is noted. If unknown, consumed amounts are expressed by household measures (e.g., a cup, a slice). Mean daily energy and macronutrient intakes in each period are calculated, e.g. using a computerized food-composition table [NEVO online version 2011/3.0; RIVM (National Institute for Public Health and the Environment)].

Participants are requested to mix 18,75 gram of a formula according to the invention or a high heat treated nutritional composition as in example 2A (reference product) containing the same amount of calories, protein and sugars (control group) twice daily in 125 ml of their soy-milk drink during the entire study.

After an adaptation period of 2 wk to the intervention products, participants fast for at least 4 h before the oral challenge with a live, but attenuated oral diarrheagenic E. coli strain as previously described (Bovee-Oudenhoven 2003; Ouwehand 2014). The E. coli strain used (E1392/75-2A) is a heat-labile/heat-stable (LT/ST) CFAll-positive variant of a previously enterotoxigenic O6:H16 LT'/ST' strain. Before (on day 21) and after (on days 1, 2, 3, 4, and 14) the diarrheagenic E. coli challenge, DNA is isolated from homogenized wet fecal samples, and diarrheagenic E. coli is quantified by qPCR as described previously (Ten Bruggencate et al 2015).

Before (on days 21 and 22) and after (on days 1, 2, 3, 4, and 14) the diarrheagenic E. coli challenge, 24-h fecal samples are collected. Fecal samples are frozen at 22°C immediately after defecation and transported to the laboratory under frozen conditions, weighed, and homogenized; aliquots are stored at 22 °C for later analyses. Diarrhea is determined by daily total fecal wet weight excretion (total fecal output), and by the percentage of fecal dry weight as determined after freeze-drying (fecal consistency).

During the entire study, participants daily report information on stool consistency by using the Bristol Stool Scale (Heaton et al 1991) and on stool frequency in an online diary. Moreover, in the online diary, participants daily record symptoms according to the validated Gastrointestinal Symptom Rating Scale (GSRS) (Svedlund et al 1988). The GSRS is a disease-specific instrument of 15 items combined into 5 symptom clusters: reflux, abdominal pain, indigestion, diarrhea, and constipation. The domain "diarrhea" consists of increased passage of stools, loose stools, and bowel urgency. The GSRS has a 7-point graded Likert-type scale where 1 = no discomfort, 2 = minor discomfort, 3 = mild discomfort, 4 = moderate discomfort, 5 = moderately severe discomfort, 6 = severe discomfort, 7 = very severe discomfort.

Blood samples (10 mL) are taken by qualified staff of a local hospital at one time point before (day 24) and at 2 time points after (days 3 and 14) the diarrheagenic E. coli challenge. Sera are prepared by low-speed centrifugation (20 min at 3000 g at 10 °C) and stored at 28°C. Concentrations of specific IgG against CFAII in sera are determined by direct ELISA as described elsewhere (Bovee-Oudenhoven et al 2003).

Fecal water extracts are prepared by adding 3.6 ml of PBS and 40µl Protease inhibitor (Sigma-Aldrich, Zwijndrecht, The Netherlands P8340) to 0,4 gram of feces, vortexing at least 30 seconds, and centrifuged at 3000x g for 10 minutes at 4°C. Fecal extracts are stored at -80°C until measurement.

To assess intestinal barrier function fecal Calprotectin (Human S100A8/S100A9), alpha-1-antitrypsin (Human SERPIN-A1) and Zonulin are measured in the fecal water extracts by ELISA according to the manufacturers' instructions (Calprotectin and alpa1-antitrypsin: R&D systems, zonulin : Alere).

In this study the nutritional composition of the invention reduces diarrhea symptoms after ETEC challenge as evidenced by a lowered frequency of stools, loose stools, gastrointestinal symptom score, and improvement of intestinal barrier as evidenced by the reduction of barrier function markers in the fecal water extracts and by the lactose/rhamnose ratio in urine.

### Example 3: Formulation of Infant formula and Growing up Milk

In table 3 an example of an Infant formula and Growing-up milk is shown. Dosage of infant formula is about 13 g / 100 mL, dosage of growing-up milk is 15 g / 100 mL

The skilled person will understand that the exact amounts of all ingredients may be adjusted for different age groups or to meet regulatory requirements.

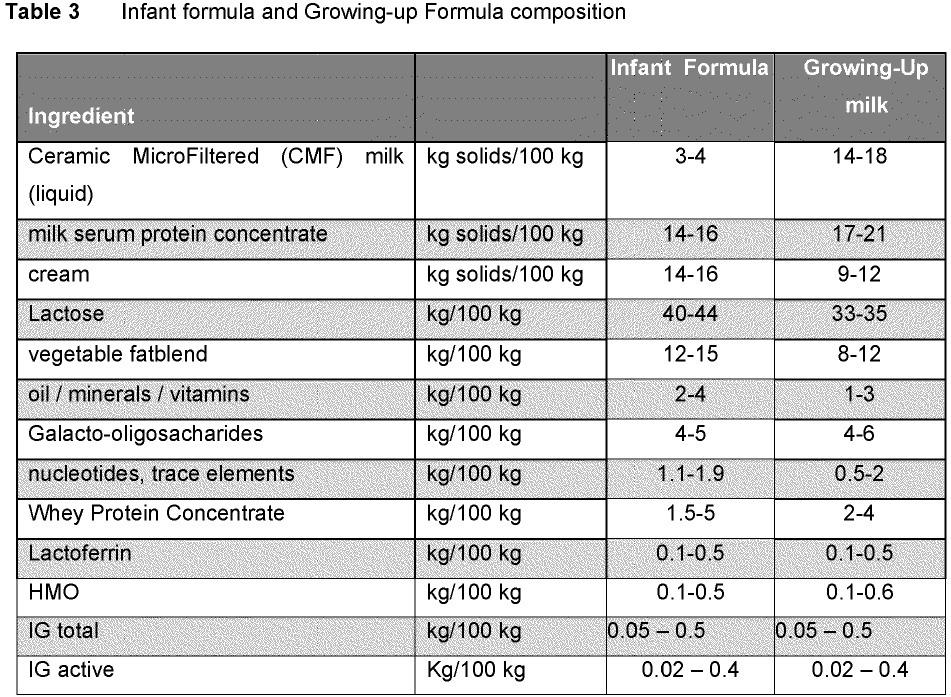

It is understood that the recipe for the infant formulation may likewise be used for a Follow-on-Formula. The infant formula or Follow-on-Formula may be powdered using techniques known in the art provided the composition is not subjected to temperatures above 80 °C.

In one embodiment, the invention relates to a composition as specified in Table 3. In another embodiment, the invention relates to a composition as specified in Table 3 wherein the galacto-oligosaccharides have been replaced with one or more other non-digestible oligosaccharides.

### REFERENCES

Bach Knudsen KE, Lærke HN, Hedemann MS, Nielsen TS, Ingerslev AK, Gundelund Nielsen DS, Theil PK, Purup S, Hald S, Schioldan AG, Marco ML, Gregersen S, Hermansen K13. Impact of Diet-Modulated Butyrate Production on Intestinal Barrier Function and Inflammation. Nutrients. 2018 Oct 13;10(10). pii: E1499. doi: 10.3390/nu10101499.
Bischoff, S. C. (2011). "Gut health": a new objective in medicine? BMC Medicine, 9(1), 24. http://doi.org/10.1186/1741-7015-9-24
Bischoff, S. C., Barbara, G., Buurman, W., Ockhuizen, T., Schulzke, J.-D., Serino, M., ... Wells, J. (2014). Intestinal permeability - a new target for disease prevention and therapy. BMC Gastroenterology, 14(1), 189. http://doi.org/10.1186/s12876-014-0189-7
Bovee-Oudenhoven IM, Lettink-Wissink ML, Van Doesburg W, Witteman BJ, Van Der Meer R. Diarrhea caused by enterotoxigenic Escherichia coli infection of humans is inhibited by dietary calcium. Gastroenterology 2003;125:469-76.
D'Souza et al: Differing roles of short chain fatty acids and GPR43 agonism in the regulation of intestinal barrier function and immune responses. Publication/Journal: PLOS One Publication date:2017 Volume/Issue: https://doi .oeg/10.1371/journal.pone.0180190 Pages: 1-22
den Hartog G, Jacobino S, Bont L, Cox L, Ulfman LH, Leusen JHW, van Neerven RJJ. Specificity and Effector Functions of Human RSV-Specific IgG from Bovine Milk. PLoS One (2014) 9:e112047. doi:10.1371/journal.pone.0112047
Hall, K. E., Proctor, D. D., Fisher, L., & Rose, S. (2005). American Gastroenterological Association future trends committee report: Effects of aging of the population on gastroenterology practice, education, and research. Gastroenterology, 129(4), 1305-1338. http://doi.org/10.1053/j.gastro.2005.06.013
Heaton KW, Ghosh S, Braddon FE. How bad are the symptoms and bowel dysfunction of patients with the irritable bowel syndrome? A prospective, controlled study with emphasis on stool form. Gut 1991;32:73-9.
Li et al Exogenous stimuli Maintain Intraepithelial Lymphocytes via aryl Hydrocarbon Receptor Activation Publication/Journal: Cell Publication date: 2011 Volume/Issue: 147 Pages: 629-640.
Godden S, McMartin S, Feirtag J, Stabel J, Bey R, Goyal S, Metzger L, Fetrow J, Wells S, Chester-Jones H. Heat-treatment of bovine colostrum. II: effects of heating duration on pathogen viability and immunoglobulin G. J Dairy Sci. 2006 Sep;89(9):3476-83.
Li SQ, Zhang HQ, Balasubramaniam VM, Lee YZ, Bomser JA, Schwartz SJ, Dunne CP. Comparison of effects of high-pressure processing and heat treatment on immunoactivity of bovine milk immunoglobulin G in enriched soymilk under equivalent microbial inactivation levels. J Agric Food Chem. 2006 Feb 8;54(3):739-46.
Man, A. L., Gicheva, N., & Nicoletti, C. (2014). The impact of ageing on the intestinal epithelial barrier and immune system. Cellular Immunology, 289(1-2), 112-118. http://doi.org/10.1016/j.cellimm.2014.04.001
Natividad JM, Agus A, Planchais J, Lamas B, Jarry AC, Martin R, Michel ML, Chong-Nguyen C, Roussel R, Straube M, Jegou S, McQuitty C, Le Gall M, da Costa G, Lecornet E, Michaudel C, Modoux M, Glodt J, Bridonneau C, Sovran B, Dupraz L, Bado A, Richard ML, Langella P, Hansel B, Launay JM, Xavier RJ, Duboc H, Sokol H. Impaired Aryl Hydrocarbon Receptor Ligand Production by the Gut Microbiota Is a Key Factor in Metabolic Syndrome. Cell Metab. 2018 Nov 6;28(5):737-749.e4. doi: 10.1016/j.cmet.2018.07.001.
Ohnuki H. and Otani H. Antigen-binding and protein G-binding abilities of immunoglobulin G in hyperimmunized cow's milk treated under various conditions 2005. 76: 283-290
Ouwehand AC, Ten Bruggencate SJ, Schonewille AJ, Alhoniemi E, Forssten SD, Bovee-Oudenhoven IM. Lactobacillus acidophilus supplementation in human subjects and their resistance to enterotoxigenic Escherichia coli infection. Br J Nutr 2014;111:465-73.
Playford RJ, Floyd DN, Macdonald CE, Calnan DP, Adenekan RO, Johnson W, Goodlad R a, Marchbank T. Bovine colostrum is a health food supplement which prevents NSAID induced gut damage. Gut (1999) 44:653-8
Svedlund J, Sjodin I, Dotevall G. GSRS-a clinical rating scale for gastrointestinal symptoms in patients with irritable bowel syndrome and peptic ulcer disease. Dig Dis Sci 1988;33:129-34.
Ten Bruggencate SJ, Girard SA, Floris-Vollenbroek EG, Bhardwaj R, Tompkins TA. The effect of a multi-strain probiotic on the resistance toward Escherichia coli challenge in a randomized, placebo-controlled, double-blind intervention study. Eur J Clin Nutr 2015;69(3):385-91.
Ten Bruggencate SJ, Frederiksen PD, Pedersen SM, Floris-Vollenbroek EG, Lucas-van de Bos E, van Hoffen E, and Wejse PL Resistance to Diarrheagenic Escherichia coli in Healthy Adults in a Randomized, Placebo-Controlled, Double-Blind Study.. J Nutr 2016;146:249-55.)
Turner, J. R. (2009). Intestinal mucosal barrier function in health and disease. Nature Reviews Immunology, 9(11), 799-809. http://doi.org/10.1038/nri2653
Valentini, L., Ramminger, S., Haas, V., Postrach, E., Werich, M., Fischer, A., ... Schulzke, J.-D. (2014). Small intestinal permeability in older adults. Physiological Reports, 2(4), e00281. http://doi.org/10.14814/phy2.281
Wang, J., Qin, J., Li, Y., Cai, Z., Li, S., Zhu, J., ... Wang, J. (2012). A metagenome-wide association study of gut microbiota in type 2 diabetes. Nature, 490(7418), 55-60. http://doi.org/10.1038/nature11450
Zhang, X., Shen, D., Fang, Z., Jie, Z., Qiu, X., Zhang, C., ... Ji, L. (2013). Human Gut Microbiota Changes Reveal the Progression of Glucose Intolerance. PLoS ONE, 8(8). http://doi.org/10.1371/journal.pone.0071108.

## Claims

1. A synthetic nutritional composition comprising:
i. ruminant milk fat wherein the milk fat is capable of activating the aryl hydrocarbon receptor (AhR) in an AhR activity assay as defined in Example 1; and
ii. a ruminant immunoglobulin in an amount of between 115 µg and 50 mg per gram of dry composition;
**characterized in that** at least 30% of the ruminant immunoglobulin is non-denatured;
and wherein the amount of milk fat is between 10 and 60 g of fat per 100 gram of formula, wherein the amount of ruminant fat is between 5 and 90 wt% as determined to the total amount of fat, and wherein the ruminant milk fat is not exposed to a temperature above 80 °C.

2. The nutritional composition of claim 1, wherein the ruminant milk fat is not exposed to a pH< 5.0.

3. The nutritional composition of claim 1 or 2, wherein the ruminant milk fat is bovine milk fat and the fat is selected from the group consisting of whole milk, cream, and anhydrous milk fat (AMF); preferably wherein the bovine is a cow.

4. The nutritional composition of any of the preceding claims wherein the composition is in a dry state comprising at least 70 wt% dry matter preferably at least 80 wt% dry matter, more preferably at least 90 wt% dry matter, most preferably at least 92 wt% dry matter.

5. The nutritional composition of any of the preceding claims wherein the immunoglobulin is selected from one or more of the group consisting of IgG, IgA, IgM, slgA, preferably wherein the immunoglobulin is IgG.

6. The nutritional composition of any of the preceding claims wherein the biological activity of the immunoglobulin is determined in an ELISA essay that discriminates between native and denatured immunoglobulin.

7. The nutritional composition of any of the preceding claims, wherein the milk fat activates the aryl hydrocarbon receptor in a Calux assay.

8. The nutritional composition of any of the preceding claims, further comprising one or more oligosaccharides selected from the group consisting of human milk oligosaccharides (HMO) and non-digestible oligosaccharides; preferably wherein the amount of HMO is between 0.01 and 5.0 gram per 100 gram of composition and / or wherein the amount of non-digestible oligosaccharide is between 0.1 and 25 gram per 100 gram of dry composition.

9. The nutritional composition of any of the preceding claims further comprising a probiotic.

10. The nutritional composition of any of the preceding claims further comprising lactoferrin, preferably bovine lactoferrin.

11. The nutritional composition of any of the preceding claims comprising
i. between 10 and 60 g of fat per 100 gram of formula, wherein the amount of ruminant fat is between 5 and 90 wt% as determined to the total amount of fat;
ii. between 115 µg - 50 mg of ruminant immunoglobulin per gram of composition (dry weight);
iii. between 10⁶ and 10⁹ cfu of probiotic per grams of dry product;
iv. between 0.1 and 25 gram of non-digestible oligosaccharide per 100 gram of formula (dry weight);
v. between 0.01 and 5.0 gram of HMO per 100 gram of dry product;
vi. between 0.1 and 20 mg of lactoferrin per gram of dry product.

12. The nutritional composition of any of the preceding claims wherein the composition is ingredient premix, preferably an early life nutrition premix.

13. The nutritional composition of any of the preceding claims, wherein the composition is selected from one or more of the group consisting of an infant formula, a follow-on formula and young child formula.

14. The nutritional composition of any anyone of claims 1 to 12, wherein the composition is a preterm formula or an adult or elderly formula.

15. Use of the nutritional composition of anyone of claims 1 to 14 in a preterm formula, an infant formula, a follow-on formula or young child formula.

## Patentansprüche

1. Synthetische Nährstoffzusammensetzung, umfassend:
i. Wiederkäuermilchfett, wobei das Milchfett in der Lage ist, den Arylkohlenwasserstoffrezeptor (AHR) in einem AHR-Aktivitätstest, wie in Beispiel 1 definiert, zu aktivieren; und
ii. ein Wiederkäuer-Immunglobulin in einer Menge zwischen 115 µg und 50 mg pro Gramm der trockenen Zusammensetzung; **Dadurch gekennzeichnet, dass** mindestens 30 % des Wiederkäuer-Immunglobulins nicht denaturiert sind;
und wobei die Menge an Milchfett zwischen 10 und 60 g Fett pro 100 Gramm der Formel beträgt, wobei die Menge an Wiederkäuerfett zwischen 5 und 90 Gew.-% beträgt, wie bestimmt auf die Gesamtmenge an Fett, und wobei das Wiederkäuermilchfett nicht einer Temperatur über 80 °C ausgesetzt ist.

2. Nährstoffzusammensetzung nach Anspruch 1, wobei das Wiederkäuermilchfett keinem pH < 5,0 ausgesetzt ist.

3. Nährstoffzusammensetzung nach Anspruch 1 oder 2, wobei das Wiederkäuermilchfett Rindermilchfett ist und das Fett aus der Gruppe ausgewählt ist, die aus Vollmilch, Sahne und wasserfreiem Milchfett (AMF) besteht; wobei das Rind vorzugsweise eine Kuh ist.

4. Nährstoffzusammensetzung nach einem der vorhergehenden Ansprüche, wobei die Zusammensetzung in einem trockenen Zustand ist, der mindestens 70 Gew.-% Trockensubstanz, vorzugsweise mindestens 80 Gew.-% Trockensubstanz, mehr bevorzugt mindestens 90 Gew.-% Trockensubstanz, am meisten bevorzugt mindestens 92 Gew.-% Trockensubstanz umfasst.

5. Nährstoffzusammensetzung nach einem der vorhergehenden Ansprüche, wobei das Immunglobulin aus einem oder mehreren der Gruppe bestehend aus IgG, IgA, IgM, slgA ausgewählt ist, wobei das Immunglobulin vorzugsweise IgG ist.

6. Nährstoffzusammensetzung nach einem der vorhergehenden Ansprüche, wobei die biologische Aktivität des Immunglobulins in einem ELISA-Essay bestimmt wird, der zwischen nativem und denaturiertem Immunglobulin unterscheidet.

7. Nährstoffzusammensetzung nach einem der vorhergehenden Ansprüche, wobei das Milchfett den Arylkohlenwasserstoffrezeptor in einem Calux-Assay aktiviert.

8. Nährstoffzusammensetzung nach einem der vorhergehenden Ansprüche, ferner umfassend ein oder mehrere Oligosaccharide, ausgewählt aus der Gruppe bestehend aus menschlichen Milcholigosacchariden (HMO) und nicht verdaulichen Oligosacchariden; vorzugsweise wobei die Menge an HMO zwischen 0,01 und 5,0 Gramm pro 100 Gramm der Zusammensetzung beträgt und / oder wobei die Menge an nicht verdaulichem Oligosaccharid zwischen 0,1 und 25 Gramm pro 100 Gramm der trockenen Zusammensetzung beträgt.

9. Nährstoffzusammensetzung nach einem der vorhergehenden Ansprüche, die ferner ein Probiotikum umfasst.

10. Nährstoffzusammensetzung nach einem der vorhergehenden Ansprüche, die ferner Lactoferrin, vorzugsweise Rinderlactoferrin, umfasst.

11. Nährstoffzusammensetzung nach einem der vorhergehenden Ansprüche, umfassend
i. zwischen 10 und 60 g Fett pro 100 g der Formel, wobei die Menge an Wiederkäuerfett zwischen 5 und 90 Gew.-%, bestimmt auf die Gesamtmenge an Fett, beträgt;
ii. Zwischen 115 µg - 50 mg Wiederkäuer-Immunglobulin pro Gramm Zusammensetzung (Trockengewicht);
iii. Zwischen 10⁶ und 10⁹ cfu Probiotikum pro Gramm Trockenprodukt;
iv. Zwischen 0,1 und 25 Gramm unverdauliches Oligosaccharid pro 100 Gramm der Formel (Trockengewicht);
v. zwischen 0,01 und 5,0 Gramm HMO pro 100 Gramm Trockenprodukt;
vi. Zwischen 0,1 und 20 mg Lactoferrin pro Gramm Trockenprodukt.

12. Nährstoffzusammensetzung nach einem der vorhergehenden Ansprüche, wobei die Zusammensetzung eine Inhaltsstoffvormischung ist, vorzugsweise eine Early Life-Nährstoffvormischung.

13. Ernährungszusammensetzung nach einem der vorhergehenden Ansprüche, wobei die Zusammensetzung aus einem oder mehreren der Gruppe ausgewählt ist, die aus einer Säuglingsnahrung, einer Folgenahrung und einer Kleinkindnahrung besteht.

14. Nährstoffzusammensetzung nach einem der Ansprüche 1 bis 12, wobei die Zusammensetzung eine Frühgeborenennahrung oder Spezialnahrung für Erwachsene oder Senioren ist.

15. Verwendung der Nährstoffzusammensetzung nach einem der Ansprüche 1 bis 14 in einer Neugeborenennahrung, einer Säuglingsnahrung, einer Folgenahrung oder einer Kleinkindnahrung.

## Revendications

1. Composition nutritionnelle synthétique comprenant :
i. de la graisse de lait de ruminant, la graisse de lait étant capable d'activer le récepteur d'hydrocarbures aryle (AhR) dans un dosage d'activité d'Ahr tel que défini dans l'exemple 1 ; et
ii. une immunoglobuline de ruminant en une quantité comprise entre 115 µg et 50 mg par gramme de composition sèche ;
**caractérisée en ce qu'**au moins 30 % de l'immunoglobuline de ruminant est non dénaturée ;
et la quantité de graisse de lait étant comprise entre 10 et 60 g de graisse par 100 grammes de formule, la quantité de graisse de ruminant étant comprise entre 5 et 90 % en poids comme déterminée par rapport à la quantité totale de graisse, et la graisse de lait de ruminant n'étant pas exposée à une température supérieure à 80 °C.

2. Composition nutritionnelle selon la revendication 1, la graisse de lait de ruminant n'étant pas exposée à un pH < 5,0.

3. Composition nutritionnelle selon la revendication 1 ou 2, la graisse de lait de ruminant étant une graisse de lait de bovin et la graisse étant choisie dans le groupe constitué par le lait entier, la crème et la graisse de lait anhydre (AMF) ; préférablement le bovin étant une vache.

4. Composition nutritionnelle selon l'une quelconque des revendications précédentes, la composition étant dans un état sec comprenant au moins 70 % en poids de matière sèche, préférablement au moins 80 % en poids de matière sèche, plus préférablement au moins 90 % en poids de matière sèche, le plus préférablement au moins 92 % en poids de matière sèche.

5. Composition nutritionnelle selon l'une quelconque des revendications précédentes, l'immunoglobuline étant choisie parmi l'une ou plusieurs du groupe constitué par IgG, IgA, IgM, sIgA, préférablement l'immunoglobuline étant IgG.

6. Composition nutritionnelle selon l'une quelconque des revendications précédentes, l'activité biologique de l'immunoglobuline étant déterminée dans un dosage ELISA qui discrimine entre une immunoglobuline native et dénaturée.

7. Composition nutritionnelle selon l'une quelconque des revendications précédentes, la graisse de lait activant le récepteur d'hydrocarbures aryle dans un dosage Calux.

8. Composition nutritionnelle selon l'une quelconque des revendications précédentes, comprenant en outre un ou plusieurs oligosaccharides choisis dans le groupe constitué par des oligosaccharides de lait humain (HMO) et des oligosaccharides non digestibles ; préférablement la quantité de HMO étant comprise entre 0,01 et 5,0 grammes par 100 grammes de composition et/ou la quantité d'oligosaccharide non digestible étant comprise entre 0,1 et 25 grammes par 100 grammes de composition sèche.

9. Composition nutritionnelle selon l'une quelconque des revendications précédentes comprenant en outre un probiotique.

10. Composition nutritionnelle selon l'une quelconque des revendications précédentes comprenant en outre de la lactoferrine, préférablement de la lactoferrine bovine.

11. Composition nutritionnelle selon l'une quelconque des revendications précédentes comprenant
i. entre 10 et 60 g de graisse par 100 grammes de formule, la quantité de graisse de ruminant étant comprise entre 5 et 90 % en poids comme déterminée par rapport à la quantité totale de graisse ;
ii. entre 115 µg et 50 mg d'immunoglobuline de ruminant par gramme de composition (poids sec) ;
iii. entre 10⁶ et 10⁹ ufc de probiotique par gramme de produit sec ;
iv. entre 0,1 et 25 grammes d'oligosaccharide non digestible par 100 grammes de formule (poids sec) ;
v. entre 0,01 et 5,0 grammes de HMO par 100 grammes de produit sec ;
vi. entre 0,1 et 20 mg de lactoferrine par gramme de produit sec.

12. Composition nutritionnelle selon l'une quelconque des revendications précédentes, la composition étant un prémélange d'ingrédients, préférablement un prémélange nutritionnel premier âge.

13. Composition nutritionnelle selon l'une quelconque des revendications précédentes, la composition étant choisie parmi l'une ou plusieurs du groupe constitué par une formule pour nourrissons, une formule de suivi et une formule pour jeunes enfants.

14. Composition nutritionnelle selon l'une quelconque des revendications 1 à 12, la composition étant une formule pour prématurés ou une formule pour adultes ou personnes âgées.

15. Utilisation de la composition nutritionnelle selon l'une quelconque des revendications 1 à 14 dans une formule pour prématurés, une formule pour nourrissons, une formule de suivi ou une formule pour jeunes enfants.
